# EUROPÄISCHE PATENTSCHRIFT

(11) **EP 0 663 996 B1**
(45) Veröffentlichungstag und Bekanntmachung des Hinweises auf die Patenterteilung: **21.05.1997**
(21) Anmeldenummer: 93921885.5
(22) Anmeldetag: 30.09.1993
(51) Int. Cl.: G01N 1/14

(54) **SCHLAMM- UND ABWASSER-PROBEENTNAHMEGERÄT UND VERFAHREN ZUR PROBEENTNAHME**
SLUDGE AND WASTE WATER SAMPLING DEVICE AND PROCESS FOR TAKING SAMPLES
APPAREIL POUR LE PRELEVEMENT D'ECHANTILLONS DE BOUES ET D'EAUX USEES ET PROCEDE DE PRELEVEMENT DE CES ECHANTILLONS

(30) Priorität: 05.10.1992 CH 3107/92; 07.09.1993 CH 2655/93
(43) Veröffentlichungstag der Anmeldung: 26.07.1995
(73) Patentinhaber: Paluschinski, Hans, CH-8311 Brütten (CH)
(72) Erfinder: Paluschinski, Hans, CH-8311 Brütten (CH)
(74) Vertreter: Feldmann, Clarence Paul
(86) Internationale Anmeldenummer: EP9302656
(87) Internationale Veröffentlichungsnummer: WO9408217

(56) Entgegenhaltungen:
- US-A- 4 024 766
- US-A- 4 037 472
- US-A- 4 077 263
- PATENT ABSTRACTS OF JAPAN vol. 5, no. 202 (P-95)(874) 22. Dezember 1981 & JP,A,56 125 644 (NIPPON BORIYUUENSU KK) 2. Oktober 1981

## Beschreibung

Die vorliegende Erfindung betrifft ein Schlamm- und Abwasser-Probeentnahmegerät gemäss Oberbegriff des Patentanspruchs 1 und ein Verfahren zur Probenentnahme unter Verwendung dieses Gerätes.

In Kläranlagen werden regelmässig Schlamm- und Abwasser-Proben entnommen und die Zusammensetzung des Schlamms, beziehungsweise des Abwassers, ständig überwacht. Diese Proben werden in den für die Kontrolle zuständigen Labors untersucht und analysiert. Dabei liegen zwei verschiedene Ausgangssituationen vor. In einem Fall muss die Probe, in diesem Fall Abwasser, aus einem Becken entnommen werden. Das Abwasser steht somit nicht unter Druck. Im anderen Fall wird die Probe aus einem Stapelbehälter oder einer Druckleitung entnommen und weist somit einen Ueberdruck auf. Diese Situation kann für Schlamm wie auch für Abwasser vorliegen.

Für die Probenentnahme für Abwasser, die nicht unter Druck stehen, sind verschiedenartige Vorrichtungen bekannt. Diese entnehmen automatisch in gewünschten Zeitabständen Proben aus den diversen Verfahrensstufen und füllen diese Proben in Probebehälter oder Probesammelbehälter ab. Alle diese bekannten Probeentnahmegeräte weisen einen Einlaufteil auf, über den das Abwasser vom Ort der Probeentnahme in einen Dosierbehälter gelangt, der mit der gewünschten Probenmenge gefüllt wird. Anschliessend läuft die Probe von dort über einen Auslaufteil in den Probebehälter ab. Das Füllen und Entleeren des Dosierbehälters kann auf unterschiedliche Art erfolgen. Einige Geräte wenden ein Verfahren an, bei dem das Abwasser mittels einer Pumpe in den Dosierbehälter befördert wird, wobei sich diese Pumpe direkt im Abwasser- oder Schlammkreislauf befindet. Derartige Geräte sind für einen Dauereinsatz ungeeignet, da die Pumpe aggressiven Bestandteilen des Abwassers oder des Schlamms direkt ausgesetzt ist und ihre Lebensdauer dadurch wesentlich beeinträchtigt wird. Besser bewährt haben sich Probeentnahmegeräte, die das Abwasser ansaugen. Hierzu wird im Dosierbehälter ein Unterdruck erzeugt, bis die gewünschte Füllmenge im Dosierbehälter erreicht worden ist.

Das zweite Verfahren stellt hohe Ansprüche an die Dichtheit des Dosierbehälters. Da der Dosierbehälter chemikalienbeständig sein, eine stabile Form aufweisen und Sichtkontakt mit der Probe gewährleisten soll, wird er bevorzugt aus Glas hergestellt. Dieser Glasbehälter wird an einer Haltevorrichtung befestigt, wobei die Dichtung mittels einem dazwischen eingeklemmten O-Ring erfolgt. Bei den bekannten Dosier-behältern ist eine Dichtung jedoch nur durch einen festen Einbau des Dosierbehälters möglich. Dieser muss jedoch auch regelmässig gründlich gereinigt werden, was bei den bekannten Systemen nur mit grossem Aufwand durchführbar ist. Zudem ist bei der Befestigung darauf zu achten, dass sich im Glas keine hohen Spannungen aufbauen, die zum Glasbruch führen können.

Die Dichtheit der bekannten Dosierbehälter reicht jedoch nicht aus, um Schlammproben zu entnehmen. Dies ist vorallem darauf zurückzuführen, dass der grosse Druck des Schlamms die auf den Dosierbehälter wirkende Kraft zu stark erhöht und dadurch die Dichtung beeinträchtigt wird.

Zur Probeentnahme von Schlamm ist lediglich ein System bekannt, bei dem eine Dosierkolbenpumpe angewendet wird. Dieses System weist aber mehrere Nachteile auf. So entstehen Fehler in der Dosierung bei Lufteinschluss oder wenn der Schlamm einen zu kleinen Ueberdruck aufweist. Eine automatische Durchspülung zwecks Reinigung ist nicht möglich. Auch eine anlässlich einer Wartung durchgeführte Reinigung ist zeitaufwendig. Aus US-A-4 037 472 ist ein Probeentnahmegerät mit einem Einlaufteil, einem Auslaufteil, einem Dosierbehälter, einer Trägerplatte und Mitteln zur Druckänderung bekannt.

Es ist bis jetzt kein Probeentnahmegerät bekannt, das einen Dosierbehälter aufweist, in dem Druckunterschiede von mehreren Atmosphären erzeugbar sind und der trotzdem auf einfache Weise und ohne Werkzeuge ausgewechselt werden kann. Ein derartiges Probeentnahmegerät zu schaffen, das sowohl für Schlamm wie auch für Abwasser einsetzbar ist, ist die Aufgabe der Erfindung.

Diese Aufgabe löst ein Schlamm- und Abwasser-Probeentnahmegerät mit den Merkmalen des Patentanspruchs 1.

Es ist eine weitere Aufgabe der Erfindung, ein Verfahren aufzuzeigen, mit dessen Hilfe Schlamm- und Abwasserproben entnommen werden können, wenn der Schlamm oder das Abwasser unter Druck anliegt.

Diese Aufgabe löst ein Verfahren unter Verwendung des erfindungsgemässen Probeentnahmegerätes mit den Merkmalen des Patentanspruches 8.

In den Zeichnungen ist ein Ausführungsbeispiel des Erfindungsgegenstandes dargestellt und in der nachfolgenden Beschreibung erläutert. Es zeigt:
- Figur 1: einen Querschnitt durch den Dosierbehälter im montierten Zustand;
- Figur 2: eine schematische Darstellung eines wesentlichen Teiles des erfindungsgemässen Probeentnahmegerätes.

Im folgenden wird zuerst das Probeentnahmegerät, beziehungsweise dessen Dosierbehälter, beschrieben. Anschliessend wird ein Verfahren zur Probeentnahme von unter Druck stehendem Schlamm oder Abwasser aufgezeigt. Dieses ist nur durchführbar, wenn ein Probeentnahmegerät verwendet wird, das wie das erfindungsgemässe Gerät selbst im gefülltem Dosierbehälter eine genügende Druckveränderung erzeugen kann.

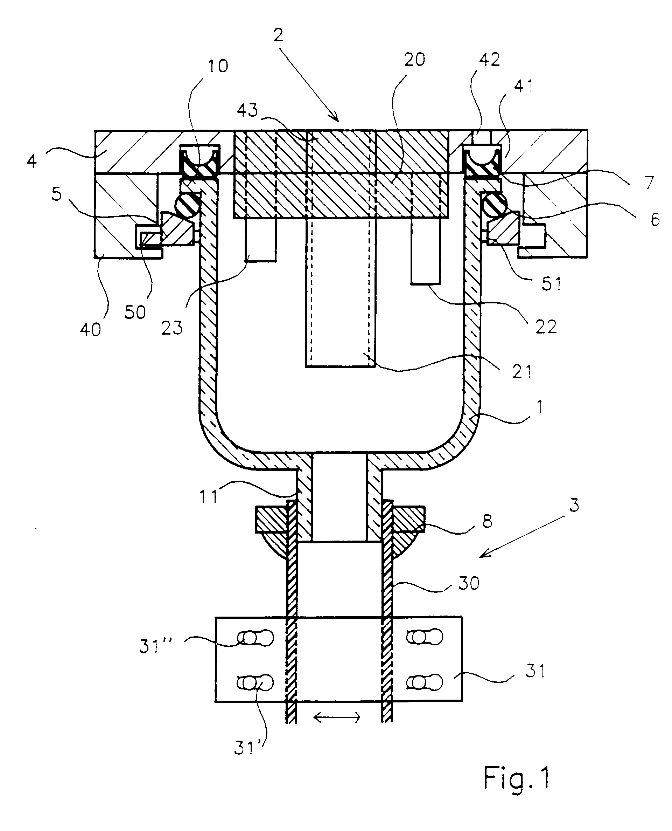

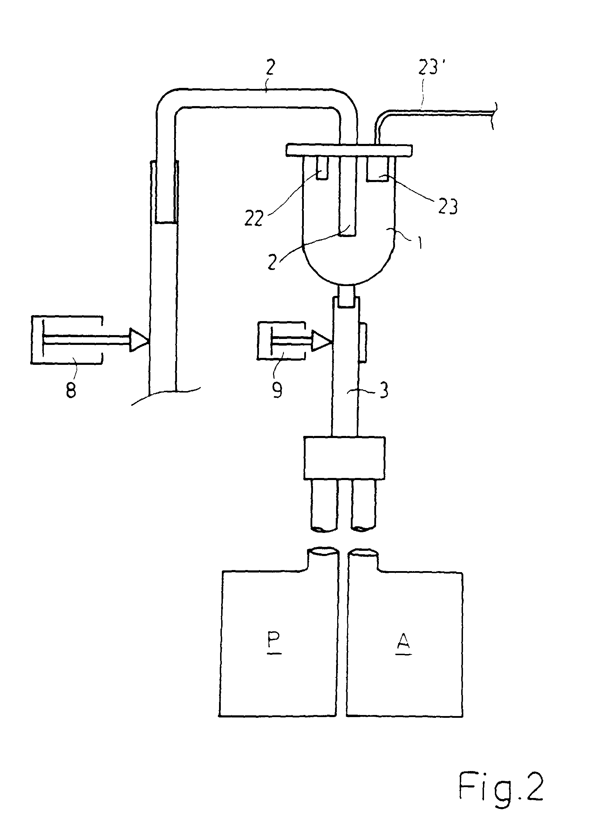

In Figur 2 ist nur der für die Erfindung wesentliche Teil des Schlamm- und Abwasser-Probeentnahmegerätes schematisch dargestellt: ein Dosierbehälter 1 mit Ein- und Auslaufteil 2,3, ein Probenbehälter P und ein Ablaufbehälter A. In Figur 1 ist der Dosierbehälter 1 mit einem Teil seiner Befestigung am Gehäuse des Gerätes und Ausschnitte des Ein- und Auslaufteils 2,3 detaillierter dargestellt. Diese Elemente befinden sich in einem Gehäuse, wobei der Dosierbehälter 1 an einer Trägerplatte 4 ankoppelbar ist, die mit dem Gehäuse des Gerätes verbunden ist. Der Einlaufteil 2 und der Auslaufteil 3 ragen teilweise aus dem Gehäuse heraus.

Der trichterförmige Dosierbehälter 1 aus Glas befindet sich zwischen dem Einlaufteil 2 und dem Auslaufteil 3 des Probeentnahmegerätes. Der Einlaufteil 2 verbindet mit einer Einlaufleitung den Entnahmeort mit dem Dosierbehälter 1. Der Auslaufteil 3 verbindet den Dosierbehälter 1 wahlweise mit einem Probenbehälter P oder einem Probensammelbehälter und einem Ablaufbehälter A oder einem Ablauf. Die Umlenkung des Auslaufteils 3 vom Probenbehälter P zum Ablaufbehälter A und umgekehrt erfolgt automatisch und wird anlässlich der Beschreibung eines mit dem Gerät anwendbaren Verfahrens näher beschrieben.

Der in der Figur 1 teilweise dargestellte Querschnitt des Einlaufteils 2 zeigt eine Modulscheibe 20. Sie befindet sich über der oberen Oeffnung des Dosierbehälters 1. In der Modulscheibe 20 befindet sich ein Einlaufrohr 21, das in den Dosierbehälter 1 hineinragt und durch das der Schlamm oder das Abwasser vom Entnahmeort in den Dosierbehälter 1 gelangt. Ebenfalls an der Modulscheibe 20 befinden sich eine Niveausonde 22 und ein Druck- und Ueberlaufventil 23. Mittels dem Druckventil 23, das an eine Druckleitung 23' gekoppelt ist, werden im Dosierbehälter 1 Druckveränderungen erzeugt, mit deren Hilfe die Einlaufleitung ausgeblasen, die Probe angesaugt oder eingelassen, die Ueberschussmenge zurückgeblasen und die Probe in den Auslaufteil abgelassen wird. Die Niveausonde 22 misst das Grenzniveau der Probenmenge im Dosierbehälter 1. Das Ueberlaufventil 23 verhindert bei defekter Niveausonde 22 ein Ueberlaufen des Dosierbehälters 1, indem es eine Ueberfüllung erkennt und durch selbstätiges Schliessen die Schlamm- bzw. Abwasser-Zufuhr stoppt.

Der Auslaufteil 3 befindet sich am Auslaufstutzen 11 des trichterförmigen Dosierbehälters 1. Ein Kunststoffschlauch 30 des Auslaufteils 3 ist durch eine Ueberwurfmutter 8 dicht mit diesem Auslaufstutzen 11 verbunden. Unterhalb der vereinfacht dargestellten Ueberwurfmutter 8 verläuft der Kunststoffschlauch 30 zwischen Gehäusewand des Gerätes und einer Gegendruckplatte 31. Die Gegendruckplatte 31 weist einseitig erweiterte Langlöcher 31' auf, mittels denen die Gegendruckplatte 31 aufgesetzt und durch Querverschiebung auf Distanzhalteschrauben 31" verriegelt und zur Entnahme des Dosierbehälters wieder gelöst werden kann. Hinter dem Kunststoffschlauch 30 befindet sich auf gleicher Höhe wie die wegnehmbare Gegendruckplatte 31 ein Ausgangsquetschventil 9. Wird der Dosierbehälter 1 mit Schlamm oder Abwasser gefüllt, so drückt dieses Ausgangsquetschventil 9 den Kunststoffschlauch 30 gegen die Gegendruckplatte 31, so dass ein dichter Abschluss des Dosierbehälters 1 erzeugt wird.
Bei der Probeentnahme von Schlamm oder Abwasser unter Druck wird zudem ein sich zwischen Dosierbehälter 1 und Einlaufleitung befindliches Eingangsquetschventil 8 mit Federvorspannung geschlossen, damit der Ueberdruck im Dosierbehälter 1 erzeugt werden kann.

Der Dosierbehälter 1 aus Glas weist an seinem oberen Ende einen Flansch 10 auf, wobei die obere Fläche des Flansches 10 eben und fluchtend mit dem Dosierbehälterrand ist. Unterhalb dieses Flansches 10 befindet sich ein Befestigungsring 5, der den Dosierbehälter 1 aussen umgibt. Der Befestigungsring 5 besitzt vorne einen leicht zugänglichen Handgriff, der in der Zeichnung nicht eingezeichnet ist. Er weist zudem auf seiner dem Dosierbehälter zugewandten Innenseite Puffer 51 aus einem weichen Material, zum Beispiel Kunststoff, auf. Dadurch wird der Dosierbehälter 1 im Befestigungsring 5 zentriert, ohne Spannungen zu erzeugen. Die obere Fläche des Befestigungsrings 5 ist leicht nach innen zum Dosierbehälter 1 hin geneigt. Auf ihr liegt ein Kunststoff-O-Ring, so dass er sich unterhalb des Flansches des Dosierbehälters 1 und oberhalb des Befestigungsringes 5 befindet. Der Befesiigungsring 5 weist auf seiner Aussenseite, vom Dosierbehälter 1 abgewandt, gleichmässig über seinem Umfang verteilte Bajonettklauen 50 auf.

Das Gegenstück zu den Bajonettklauen bilden die Bajonettlager 40 der Trägerplatte 4. Die Trägerplatte 4 befindet sich oberhalb des Dosierbehälters 1. Die Bajonettlager 40 sind seitlich und nach unten ragend an ihrem Umfang angeordnet und umfassen den Dosierbehälter 1 teilweise. Die Trägerplatte 4 weist in ihrem Zentrum eine kreisförmige Oeffnung 43 auf, die vom Einlaufrohr 21 des Einlaufteils 2 durchsetzt wird. In der Trägerplatte 4 über dem Flansch 10 des Dosierbehälters 1 ist eine ringförmige Aufnahme 41 mit demselben Radius wie der Radius des Flansches vorhanden. Diese Aufnahme 41 kann sich auch in einer separaten Platte befinden, die an der Trägerplatte 4 befestigt ist. In dieser Aufnahme 41 befindet sich ein Profildichtring 7 mit einem u-förmigen Querschnitt. Die geschlossene Seite des U-Profils zeigt in Richtung des Flansches 10 des Dosierbehälters 1. Der Profildichtring 7 ist so bemessen, dass er sich unter Vorspannung in der Aufnahme 41 der Trägerplatte 4 befindet. Zudem weist die Aufnahme 41 auf der vom Flansch 10 abgewandten Seite an einer oder mehreren Stellen eine durch die Trägerplatte 4 durchgehende Bohrung 42 auf. Durch diese Bohrung kann Luft in den zwischen Aufnahme 41 und Profildichtring 7 entstandenen Hohlraum gepresst werden.

Im Betriebszustand des Probeentnahmegerätes wird durch den oben beschriebenen Aufbau der Dosierbehälter automatisch dicht abgeschlossen, wie im folgenden näher dargelegt wird. Der Dosierbehälter 1 ist einerseits mechanisch mit einem Bajonettverschluss durch den Befestigungsring 5 an der Trägerplatte 4 befestigt. Der Dosierbehälter ist trotzdem wegen dem O-Ring 6 und den Puffern 51 spannungsfrei und zentriert gehalten. Dies hat jedoch zur Folge, dass auf den Dosierbehälter 1 je nach Füllgewicht und vorallem je nach Druckverhältnis im Dosierbehälter verschiedene Kräfte wirken, so dass er den O-Ring unterschiedlich stark zusammendrückt und sich verschieden tief auf den Befestigungsring 5 absenkt. Die Dichtheit des Behälters ist aber trotzdem durch den Profildichtring 7 gewährleistet. Wenn im Betriebszustand das pneumatische Quetschventil den Auslaufteil 3 schliesst, wird der Profildichtring 7 über die Bohrung 42 in der Trägerplatte 4 ebenfalls pneumatisch auf den Flansch 10 des Dosierbehälters 1 gedrückt. So liegt bei Absenkung des Dosierbehälters 1 der Profildichtring 7 stets dichtend auf dem Flansch 10 des Dosierbehälters 1. Die Pneumatik des Profildichtrings 7 kann mit der Pneumatik des Quetschventils gekoppelt sein. Ebenso kann sie mit den Druckveränderungsmitteln, die die Druckveränderung im Dosierbehälter erzeugen, verbunden sein oder auch als einzelnes Teil angesteuert werden.

Will man den Dosierbehälter 1 vom Gerät entfernen, um ihn auszuwechseln oder zu reinigen, sind nur wenige Handgriffe notwendig. Die Gegendruckplatte 31 wird zur Seite geschoben, so dass die Distanzschrauben 31" an der erweiterten Stelle der Langlöcher 31' liegen und die Gegendruckplatte 31 von der Befestigung entfernt werden kann. Der Befestigungsring 5 wird zusammen mit dem Dosierbehälter 1 mit Hilfe des Haltegriffes aus dem Bajonettverschluss gedreht. Da die Pneumatik des Profildichtrings 7 nicht mehr im Betrieb ist, lässt sich der Dosierbehälter 1 zusammen mit dem Befestigungsring 5 und dem Auslaufteil 3 leicht aus dem Probeentnahmegerät entfernen und ebenso leicht wieder einsetzen.

Ein weiterer Vorteil dieses Aufbaus ist, dass sich der Profildichtring 7 weit vom Einlaufrohr 21 und vom Ueberlaufventil 23 entfernt befindet. Eine Verschmutzung des Profildichtrings 7 durch Schlamm- oder Abwasser-Spritzer ist unwahrscheinlich. Die Dichtheit kann somit auch nicht durch Verunreinigung des Profildichtrings 7 beeinträchtigt werden.

Das erfindungsgemässe Probeentnahmegerät kann somit zur Durchführung des eingangs beschriebenen Verfahrens eingesetzt werden. Das heisst, es kann zum Ansaugen von drucklosem. Abwasser eingesetzt werden. Es arbeitet jedoch effizienter als die bekannten Geräte, da grössere Druckunterschiede erzielt werden können. Ebenso ist es anlässlich einer Wartung leichter zu reinigen, da der Dosierbehälter mit wenigen Handgriffen aus seiner Verankerung gelöst werden kann. Da jedoch Druckunterschiede von mehreren Atmosphären erzielt werden können, kann der Dosierbehälter auch unter Verwendung dieser Druckunterschiede automatisch mit Wasser ausgespült werden. Hierfür ist ein Wasserzulauf im Einlaufteil oder im Dosierbehälter vorzusehen. Der Ablauf erfolgt über den Auslaufteil.

Dank der optimalen Dichtung des Dosierbehälters können aber auch Schlammproben entnommen werden.

Zudem kann nun ein neues Verfahren angewendet werden, bei dem von unter Druck stehendem Schlamm oder Abwasser automatisch Proben entnommen werden. Dieses Verfahren wird nun im folgenden beschrieben.
Der Einlaufteil 2 ist in diesem Fall mit einem Stapelbehälter oder einer Druckleitung verbunden. Der Auslaufteil 3 ist wie oben beschrieben wahlweise mit einem Proben/Probensammelbehälter und einem Ablauf/Ablaufbehälter verbunden.
Zu Beginn des Verfahrens wird das Eingangsquetschventil 8 und das Ausgangsquetschventil 9 geschlossen, so dass Ein- und Auslaufteil 2,3 unterbrochen sind. Das Eingangsquetschventil 8 ist dabei unter Federspannung drucklos geschlossen. Im Dosierbehälter 1 wird nun über das Ventil 23 ein Ueberdruck gegenüber dem im Einlaufteil herrschenden Schlamm- beziehungsweise Abwasserdruck aufgebaut. Anschliessend wird das Eingangsquetschventil 8 und somit der Einlaufteil geöffnet, das heisst, der Druck im Dosierbehälter wird gegenüber der Entnahmestelle entspannt. Der Ueberdruck im Dosierbehälter wird nun reduziert, bis er maximal den anliegenden Druck des Abwassers beziehungsweise des Schlamms aufweist. Der Druck im Dosierbehälter wird langsam, bevorzugterweise stufenweise, weiter reduziert. Dadurch dringt Schlamm beziehungsweise Abwasser durch den Einlaufteil in den Dosierbehälter ein. Nach Erreichen eines vordefinierten Volumens im Dosierbehälter wird der Druck im Dosierbehälter wieder erhöht, damit der nachfolgende Schlamm oder das nachfolgende Abwasser wieder soweit durch den Einlaufteil zur Entnahmestelle zurückgefördert wird, bis das Probenvolumen erreicht ist. Daraufhin wird das Eingangsquetschventil geschlossen. Im Dosierbehälter befindet sich nur die vordefinierte Probenmenge. Nun wird das Ausgangsquetschventil 9 geöffnet und die entnommene Probe wird vom Dosierbehälter in den Probenbehälter P abgegeben.

Dieses Verfahren kann durch verschiedene Schritte verfeinert werden.
In einer Variante wird vor der eigentlichen Probeentnahme der als Totvolumen bezeichnete Inhalt in den Zuleitungen unter gleichzeitigem Spülen in den Ablauf abgeleitet.
In einer Variante des Verfahrens wird, nachdem die gewünschte Probenmenge im Dosierbehälter abgemessen wurde, diese nicht in den Probenbehälter abgelegt. Sie gelangt vielmehr durch Oeffnen des Ausgangsquetschventils 9 ungenutzt und unausgewertet in einen Ablaufbehälter A oder direkt in einen hierfür vorgesehenen Ablauf. Dadurch wird verhindert, dass eine die Wirklichkeit verfälschende Probe aus dem Anfang einer Druckleitung oder aus einem Randgebiet des Stapelbehälters ausgewertet wird. Der Dosierbehälter und die Ablaufleitung wird anschliessend automatisch gespült. Als Spülmittel wird vorzugsweise Wasser verwendet. Das Spülmittel wird wie die nicht verwendeten Proben in den Ablaufbehälter abgeleitet. Diese Schritte können je nach Anwendungsort mehrmals automatisch durchgeführt werden. Anschliessend wird die zur Auswertung bestimmte Probe genommen. Der Auslaufteil wird nun zum Probenbehälter oder Probensammelbehälter umgelenkt und die Probe durch Oeffnen des Ausgangsquetschventils in diesen Probenbehälter abgegeben. Anschliessend wird der Dosierbehälter noch mindestens einmal mit dem Spülmittel gereinigt. Dies kann zur besseren Reinigung unter Ueberdruck erfolgen. Der Dosierbehälter ist somit gereinigt und das Probeentnahmegerät ist für den nächsten Einsatz bereit. Ist eine gründlichere Reinigung notwendig, so wird der Dosierbehälter auf die oben beschriebene, einfache Weise aus seiner Befestigung gelöst.

Im Dosierbehälter befindet sich eine Niveausonde 22. Mittels dieser wird die gewünschte Probenmenge bestimmt. In einer Variante des Verfahrens wird jedoch eine maximale Zeitspanne vorgegeben, während der Schlamm oder Abwasser in den Dosierbehälter eindringen kann. Wird die gewünschte Probenmenge vor Ablauf der Zeitspanne erreicht, was mittels der Niveausonde festgestellt wird, so wird der nachfolgende Schlamm oder das nachfolgende Abwasser mittels Ueberdruck zurückgefördert. Die Dosierung erfolgt also mittels Rückförderung des überschüssigen Materiales.

Ist die gewünschte Probenmenge nach Ablauf der Zeitspanne aber noch nicht erreicht, so wird nun im Dosierbehälter ein Unterdruck gegenüber dem anliegenden Schlamm- beziehungsweise Abwasserdruck erzeugt. Der Schlamm oder das Abwasser wird nun in den Dosierbehälter hineingesogen. Dies erfolgt bis zur Erreichung des durch die Niveausonde festgelegten Niveaus. Anschliessend wird das Eingangsquetschventil geschlossen und die entnommene Probe in den Probenbehälter abgegeben. Diese Variante des Verfahrens ist vorallem sinnvoll, wenn der Schlamm oder das Abwasser nur einen schwachen Ueberdruck aufweisen. Somit kann die Probe trotzdem innerhalb kurzer Zeit entnommen werden und Lufteinschlüsse oder zu kleine Probenmengen werden verhindert.

## Patentansprüche

1. Schlamm- und Abwasser-Probeentnahmegerät, das einen Einlaufteil (2) und einen Auslaufteil (3) mit dazwischenbefindlichem, austauschbarem und an einer Trägerplatte (4) gehaltenen Dosierbehälter (1) und Mittel zur Druckveränderung im Dosierbehälter (1) aufweist, dadurch gekennzeichnet,
dass der Dosierbehälter (1) mittels einem Befestigungsring (5) mechanisch an der Trägerplatte (4) ankoppelbar ist,
wobei der Dosierbehälter (1) mindestens einen nach aussen gerichteten Flansch (10) aufweist, unter dem sich ein Kunststoffring (6) befindet, der im angekoppelten Zustand des Dosierbehälters (1) auf dem Befestigungsring (5) aufliegt,
und dass im angekoppelten Zustand des Dosierbehälters (1) sich ein Profildichtring (7) über dessen Flansch (10) befindet, der pneumatisch dichtend auf den Dosierbehälter (1) pressbar ist.

2. Schlamm- und Abwasser-Probeentnahmegerät nach Anspruch 1, dadurch gekennzeichnet, dass der Befestigungsring (5) mittels Bajonettverschluss an der Trägerplatte (4) befestigbar ist.

3. Schlamm- und Abwasser-Probeentnahmegerät nach Anspruch 1, dadurch gekennzeichnet, dass der Profildichtring (7) ein U-Profil aufweist und mit seiner offenen Seite in einer ringförmigen Aufnahme (41) in der Trägerplatte (4) liegt, wobei die Aufnahme (41) über Bohrungen (42) mit der Pneumatik kommuniziert.

4. Schlamm- und Abwasser-Probeentnahmegerät nach Anspruch 1, dadurch gekennzeichnet, dass der Kunststoffring (6) ein O-Ring ist, der auf einer geneigten Fläche des Befestigungsrings (5) aufliegt, so dass er durch die auf den Dosierbehälter (1) wirkende Kraft zusammendrückbar ist.

5. Schlamm- und Abwasser-Probeentnahmegerät nach Anspruch 1, dadurch gekennzeichnet, dass der Befestigungsring (5) auf der Dosierbehälterseite Puffer (51) aus weichem Material aufweist.

6. Schlamm- und Abwasser-Probeentnahmegerät nach Anspruch 1, dadurch gekennzeichnet, dass die Pneumatik des Profildichtrings (7) mit den Druckveränderungsmitteln des Dosierbehälters (1) gekoppelt ist.

7. Schlamm- und Abwasser-Probeentnahmegerät nach Anspruch 1, dadurch gekennzeichnet, dass der Auslaufteil (3) einen Kunststoffschlauch (30) umfasst, der hinter einer formschlüssig gehaltenen Gegendruckplatte (31) im Bereich eines Quetschventils (9) durchgeführt ist.

8. Verfahren zur Probeentnahme von unter Druck stehendem Schlamm oder Abwasser unter Verwendung des Gerätes nach Anspruch 1, wobei der Einlaufteil und der Auslaufteil je mittels einem Verschlussmittel verschliessbar sind,
dadurch gekennzeichnet, dass
a) im Dosierbehälter ein Ueberdruck gegenüber dem anliegenden Schlamm- beziehungsweise Abwasserdruck aufgebaut wird, wobei Einlaufteil und Auslaufteil mittels den Verschliessmitteln geschlossen sind,
b) dass der Einlaufteil geöffnet wird,
c) dass der Ueberdruck im Dosierbehälter gegenüber dem Schlamm- beziehungsweise Abwasserdruck reduziert wird, so dass Schlamm beziehungsweise Abwasser in den Dosierbehälter eindringt,
d) dass nach Erreichen des vordefinierten Probenvolumens der nachfolgende Schlamm oder das nachfolgende Abwasser unter Ueberdruck gegenüber dem Schlamm- bzw. Abwasserdruck durch den Einlaufteil zurückgefördert wird,
e) dass der Auslassteil geöffnet wird und
f) dass die entnommene Probe vom Dosierbehälter in einen Probenbehälter oder einem Probensammelbehälter abgegeben wird.

9. Verfahren nach Anspruch 8, dadurch gekennzeichnet,
dass nach den Schritten a) bis e) die entnommene Probe abgelassen wird,
dass anschliessend der Dosierbehälter gespült wird,
dass obige Schritte mindestens einmal durchgeführt werden und
dass anschliessend die Schritte a) bis f) durchgeführt werden.

10. Verfahren nach einem der Ansprüche 8 oder 9, dadurch gekennzeichnet, dass nach dem Ablegen der Probe in den Probenbehälter der Dosierbehälter und die Ablaufleitung mindestens einmal unter Ueberdruck gespült wird.

11. Verfahren nach einem der Ansprüche 9 bis 10, dadurch gekennzeichnet, dass zum Spülen Wasser verwendet wird.

12. Verfahren nach Anspruch 8, dadurch gekennzeichnet, dass die im Dosierbehälter befindliche Probenmenge mittels einer Niveausonde bestimmt wird.

13. Verfahren nach Anspruch 12, dadurch gekennzeichnet, dass das Eindringen des Schlamms beziehungsweise des Abwassers in Schritt c) zeitlich beschränkt ist und
dass, falls nach Ablauf dieser Zeitspanne die durch die Niveausonde bestimmte Probenmenge noch nicht erreicht ist, im Dosierbehälter der Druck gegenüber dem Schlamm- bzw. Abwasserdruck stärker reduziert wird, so dass der Schlamm bzw. das Abwasser bis zur Erreichung der vordefinierten Probenmenge in den Dosierbehälter hineingesogen wird.

## Claims

1. A sludge and sewage sampling device having an inlet part (2) and an outlet part (3) with an interposed, replaceable dosing container (1) held on a support plate (4) and means for changing the pressure in the dosing container (1), characterised in that the dosing container (1) mechanically couples to the support plate (4) by means of a ring fastener (5), the dosing container (1) having at least one outwardly directed flange (10) below which is located a plastics ring (6) which rests on the ring fastener (5) when the dosing container (1) is in the coupled condition, and that in the coupled condition of the dosing container (1) a profiled gasket (7) is located above the flange (10) thereof and can be pressed in pneumatically sealing manner onto the dosing container (1).

2. A sludge and sewage sampling device according to claim 1, characterised in that the ring fastener (5) is securable to the support plate (4) by means of a bayonet fastening.

3. A sludge and sewage sampling device according to claim 1, characterised in that the profiled gasket (7) has a U-profile and is located with its open side in an annular receptacle (41) in the support plate (4), the receptacle (41) communicating via bores (42) with the pneumatics.

4. A sludge and sewage sampling device according to claim 1, characterised in that the plastics ring (6) is an O-ring, which rests on an inclined surface of the ring fastener (5) so that it can be compressed by the force acting on the dosing container (1).

5. A sludge and sewage sampling device according to claim 1, characterised in that the ring fastener (5) has buffers of soft material (51) on the side of the dosing container.

6. A sludge and sewage sampling device according to claim 1, characterised in that the pneumatics of the profiled gasket (7) are coupled to the pressure modifying means of the dosing container (1).

7. A sludge and sewage sampling device according to claim 1, characterised in that the outlet part (3) incorporates a plastics hose (30) passing through behind a positively retained pressure plate (31) in the vicinity of a pinch valve (9).

8. A method for taking samples from pressurised sludge or sewage using the device according to claim 1, the inlet part and the outlet part in each case being closable by means of a closing means,
characterised in that
a) an overpressure is built up in the dosing container with respect to the applied sludge or sewage pressure, the inlet part and outlet part being closed by the closing means,
b) the inlet part is opened,
c) the overpressure in the dosing container is reduced compared with the eludge or sewage pressure, so that sludge or sewage penetrates the dosing container,
d) on reaching a predefined sample volume the following sludge or sewage is conveyed back through the inlet part under an overpressure compared with the sludge or sewage pressure,
e) the outlet part is opened, and
f) the sample removed from the dosing container is fed into a sample container or a sample collecting container.

9. A method according to claim 8, characterised in that following stages a) to e) the removed sample is drained off, the dosing container is subsequently washed, the above stages are performed at least once and then stages a) to f) are performed.

10. A method according to one of claims 8 or 9, characterised in that, after placing the sample in the sample container, the dosing container and drain line are washed at least once under an overpressure.

11. A method according to one of claims 9 or 10, characterised in that water is used for washing purposes.

12. A method according to claim 8, characterised in that the sample quantity in the dosing container is determined by means of a level probe.

13. A method according to claim 12, characterised in that the penetration of sludge or sewage in stage c) is time-limited and that, if at the end of the time interval the sample quantity determined by the level probe has still not been reached, the pressure in the dosing container is reduced to a greater extent compared with the sludge or sewage pressure, so that the sewage or sludge can be sucked into the dosing container until the predefined sample quantity is reached.

## Revendications

1. Appareil pour le prélèvement d'échantillons de boues et d'eaux usées, qui présente une partie d'entrée (2) et une partie de sortie (3), avec un récipient de dosage (1) intercalé de façon amovible et fixé sur une plaque support (4) et un moyen pour modifier la pression dans le récipient de dosage (1), caractérisé en ce
que le récipient de dosage (1) peut être fixé mécaniquement à la plaque support (4) au moyen d'une bague de fixation (5),
tandis que le récipient de dosage (1) présente au moins une collerette (10) tournée vers l'extérieur, sous laquelle se trouve un anneau en matière plastique (6) qui, lorsque le récipient de dosage (1) est accouplé, repose sur la bague de fixation,
et que, lorsque le récipient de dosage (1) est accouplé, est présent sur sa collerette (10) un anneau d'étanchéité profilé (7) qui peut être comprimé pneumatiquement pour assurer l'étanchéité sur le récipient de dosage (1).

2. Appareil pour le prélèvement d'échantillons de boues et d'eaux usées selon la revendication 1, caractérisé en ce que la bague de fixation (5) peut être fixée à la plaque support (4) au moyen d'un emboîtement à baionnette.

3. Appareil pour le prélèvement d'échantillons de boues et d'eaux usées selon la revendication 1, caractérisé en ce que l'anneau d'étanchéité profilé (7) présente un profil en U et repose avec sa face ouverte dans un logement annulaire (41) dans la plaque support (4), tandis que le logement (41) communique par des trous (42) avec le système pneumatique.

4. Appareil pour le prélèvement d'échantillons de boues et d'eaux usées selon la revendication 1, caractérisé en ce que l'anneau de matière plastique (6) est un anneau circulaire qui repose sur une surface inclinée de la bague de fixation (5), de telle sorte qu'il puisse être comprimé par la force agissant sur le récipient de dosage (1).

5. Appareil pour le prélèvement d'échantillons de boues et d'eaux usées selon la revendication 1, caractérisé en ce que la bague de fixation (5) présente sur la face tournée du côté du récipient de dosage des tampons (51) en matière souple.

6. Appareil pour le prélèvement d'échantillons de boues et d'eaux usées selon la revendication 1, caractérisé en ce que le système pneumatique de l'anneau d'étanchéité profilé (7) est accouplé aux moyens de modification de la pression du récipient de dosage (1).

7. Appareil pour le prélèvement d'échantillons de boues et d'eaux usées selon la revendication 1, caractérisé en ce que la partie de sortie (3) comprend un tuyau en matière plastique (30) qui passe derrière une plaque de contre-pression (31) maintenue avec engagement positif dans la zone d'une soupape d'écrasement (9).

8. Procédé pour le prélèvement d'échantillons de boues ou d'eaux usées se trouvant sous pression, avec utilisation de l'appareil selon la revendication 1, tandis que la partie d'entrée et la partie de sortie peuvent être chacune obturées par l'intermédiaire d'un moyen de fermeture, caractérisé en ce que
a) dans le récipient de dosage est établie une surpression par rapport à la pression respectivement des boues ou des eaux usées adjacentes, tandis que la partie d'entrée et la partie de sortie sont fermées par l'intermédiaire des moyens d'obturation,
b) que la partie d'entrée est ouverte,
c) que la surpression dans le récipient de dosage par rapport à la pression respectivement des boues ou des eaux usées est réduite, de telle sorte que respectivement les boues ou les eaux usées pénètrent dans le récipient de dosage,
d) que, une fois atteint le volume prédéfini d'échantillon, les boues ou les eaux usées subséquentes sont refoulées sous surpression par rapport à la pression respectivement des boues ou des eaux usées à travers la partie d'entrée,
e) que la partie de sortie est ouverte et
f) que l'échantillon prélevé du récipient de dosage est envoyé dans un récipient d'échantillonnage ou dans un récipient de recueil d'échantillons.

9. Procédé selon ia revendication 8, caractérisé en ce que, après les étapes a) à e), l'échantillon prélevé est soutiré,
que le récipient de dosage est ensuite rincé,
que les étapes susdites sont effectuées au moins une fois, que les étapes a) à f) sont ensuite mises en oeuvre.

10. Procédé selon l'une des revendications 8 ou 9, caractérisé en ce que, après le dépôt de l'échantillon dans le récipient d'échantillonnage le récipient de dosage et la conduite de décharge sont rincés sous surpression au moins une fois.

11. Procédé selon l'une des revendications 9 à 10, caractérisé en ce qu'on utilise de l'eau pour le rinçage.

12. Procédé selon la revendication 8, caractérisé en ce que la quantité d'échantillon se trouvant dans le récipient de dosage est déterminée au moyen d'une sonde de niveau.

13. Procédé selon la revendication 12, caractérisé en ce que l'introduction respectivement des boues ou des eaux usées dans l'étape c) est limitée dans le temps et que, dans le cas où une fois cet intervalle de temps écoulé la quantité d'échantillon déterminée par la sonde de niveau n'est par encore atteinte, on réduit plus fortement la pression par rapport à la pression respectivement des boues ou des eaux usées dans le récipient de dosage, de telle sorte que respectivement les boues ou les eaux usées soient aspirées jusqu'à ce que soit atteinte la quantité d'échantillon prédéfinie dans le récipient de dosage.
